# FASCICULE DE BREVET EUROPEEN

(11) **EP 0 655 235 B1**
(45) Date de publication et mention de la délivrance du brevet: **07.08.1996**
(21) Numéro de dépôt: 94402413.2
(22) Date de dépôt: 26.10.1994
(51) Int. Cl.: A61K 7/48, A61K 7/40

(54) **Composition amincissante**
Schlankmachende Zusammensetzung
Slimming composition

(30) Priorité: 26.11.1993 FR 9314156
(43) Date de publication de la demande: 31.05.1995
(73) Titulaire: L'OREAL, F-75008 Paris (FR)
(72) Inventeur: Soudant, Etienne, F-94260 Fresnes (FR); Nadaud, Jean-François, F-92140 Clamart (FR)
(74) Mandataire: Lhoste, Catherine

(56) Documents cités:
- EP-A- 0 371 844
- EP-A- 0 493 151
- WO-A-92/11838
- FR-A- 2 273 514
- FR-A- 2 369 840
- FR-A- 2 679 770
- PATENT ABSTRACTS OF JAPAN vol. 8, no. 134 (C-230) (1571) 21 Juin 1984 & JP-A-59 044 313 (YAKULT HONSHA K.K.) 12 Mars 1984

## Description

La présente invention concerne un procédé de traitement et/ou de prévention visant plus particulièrement à diminuer ou à lutter contre les problèmes d'adiposité, et ceci dans le but notamment d'obtenir un effet cosmétique et/ou dermatologique d'amincissement généralisé ou localisé sur le corps humain ou animal.

Elle concerne également diverses compositions, cosmétiques, dermatologiques et/ou médicamenteuses, destinées notamment à la mise en oeuvre dudit procédé, ainsi que leurs différentes utilisations.

L'adiposité (ou excès de graisse dans le tissu cellulaire sous-cutané) peut avoir de nombreuses causes plus ou moins complexes, plus ou moins connues ou plus ou moins comprises.

Certaines cellules de la peau, appelées adipocytes, contiennent des quantités variables de graisses sous la forme de triglycérides, ces triglycérides étant synthétisés *in vivo* par les adipocytes eux-mêmes, selon des réactions de type enzymatique (lipogénèse), à partir des acides gras libres et du glucose (après dégradation de ce dernier en glycérol) contenus dans l'organisme et apportés à celui-ci par l'intermédiaire de certains aliments. Or, parallèlement, les triglycérides ainsi formés, puis stockés, dans les cellules adipocytes peuvent également se redécomposer, toujours sous l'action d'enzymes spécifiques (lipolyse) contenues dans ces mêmes cellules, en libérant cette fois des acides gras d'une part et du glycérol et/ou des mono- et/ou des di-esters du glycérol d'autre part. Les acides gras ainsi relargués peuvent alors soit diffuser dans l'organisme pour y être consommés ou transformés de différentes façons, soit être recaptés (aussitôt ou un peu plus tard) par les adipocytes pour générer à nouveau des triglycérides par lipogénèse.

Si, pour des raisons diverses (nourriture trop riche, inactivité, vieillissement et autres), un déséquilibre substantiel s'installe dans l'organisme entre la lipogénèse (formation de triglycérides par réaction enzymatique entre des acides gras et le glycérol provenant du glucose) et la lipolyse (décomposition enzymatique de triglycérides en acides gras et glycérol), c'est à dire plus précisément si les quantités de graisses formées par lipogénése deviennent notablement et constamment supérieures à celles qui sont éliminées par lipolyse, il se produit alors dans les adipocytes une accumulation de triglycérides qui, si elle devient excessive, peut se traduire progressivement par l'apparition d'une peau épaisse, à surface souvent irrégulière ("peau d'orange") et de consistance plus ou moins flasque ou gélatineuse, donnant finalement à la silhouette un aspect général disgracieux pouvant évoluer entre la simple surcharge locale (lipodismorphie), en passant par l'embonpoint certain, et enfin la réelle obésité.

Or, compte tenu notamment du profond inconfort tant physique qu'esthétique, et parfois psychologique, qu'elle occasionne auprès des individus qui en sont atteints, en particulier chez les femmes, I'adiposité constitue de nos jours une affection de moins en moins bien supportée ou acceptée.

Des méthodes ont certes déja été proposées en vue de traiter l'adiposité, mais parmi celles-ci, seules en fait les méthodes reposant sur des traitements chirurgicaux, tels que la liposuccion, permettent actuellement d'obtenir des résultats véritablement satisfaisants. Toutefois, de tels traitements présentent bien évidemment comme inconvénient majeur de nécessiter la mise en oeuvre sur le corps humain ou animal d'opérations invasives par nature délicates, non sans risques et souvent coûteuses.

Il existe donc aujourd'hui dans l'état de l'art un fort besoin quant à pouvoir disposer d'un procédé de traitement cosmétique et/ou non thérapeutique "doux", de type non chirurgical, et permettant de lutter efficacement contre l'adiposité humaine ou animale, et ceci en vue notamment d'obtenir un effet général, ou au contraire localisé, d'amincissement et/ou d'affinement de la peau ou de la silhouette.

La présente invention vise justement à la satisfaction d'un tel besoin.

Ainsi, à la suite d'importantes recherches menées sur la question et au cours desquelles la Demanderesse a été amenée, dans sa démarche inventive, à recenser, analyser et interpréter les multiples causes, facteurs et mécanismes, pour partie connus en soi, liés à l'adiposité, il a maintenant été trouvé, de façon tout à fait inattendue et surprenante, que l'objectif ci-dessus pouvait être atteint au moyen d'un nouveau procédé de traitement dont le principe général de base consiste essentiellement à limiter, ou à inhiber totalement, la captation du glucose par les cellules dites adipocytes contenues dans la peau. Ainsi, en limitant ou en empêchant la diffusion, à l'intérieur des adipocytes, du glucose extra-cellulaire contenu dans l'organisme (c'est à dire, finalement, en "tarissant" la source de glycérol indispensable au stockage des matières grasses), il s'est avéré possible de diminuer de manière substantielle, dans ces mêmes adipocytes, la formation des triglycérides qui sont normalement et naturellement générés par lipogénèse à partir des acides gras libres provenant soit de l'alimentation soit de la lipolyse elle-même. Par ailleurs, d'un point de vue pratique, il a été trouvé que l'effet technique recherché pouvait être obtenu, et ceci avec des résultats particulièrement remarquables, en apportant à l'organisme, en particulier aux cellules de la peau, certaines substances spécifiques non cytotoxiques et capables d'inhiber de manière efficace la captation du glucose par les adipocytes. Enfin, il a été trouvé que les effets ci-dessus pouvaient encore être renforcés lorsque l'utilisation desdits inhibiteurs de la captation du glucose était en outre couplée ou cumulée avec l'utilisation d'au moins un composé capable de stimuler ou activer la lipolyse (effet cumulé).

Toutes ces découvertes sont à la base de la présente invention.

Ainsi, selon un premier aspect de la présente invention, il est maintenant proposé un procédé de traitement *in vivo* non thérapeutique en vue de lutter contre l'adiposité et obtenir ainsi un effet d'amincissement de la peau, ledit procédé étant caractérisé par le fait qu'il consiste à administrer à l'organisme, par voie topique et/ou systémique, au moins une substance non soufrée capable de imiter ou d'inhiber la captation du glucose par les adipocytes, choisie, seule ou en mélanges, parmi la sérine ou ses polymères correspondants, la rutine ou ses polymères correspondants, et les céramides.

Ces produits se sont en effet révélés comme particulièrement efficaces dans le procédé selon l'invention pour diminuer le taux des graisses dans la peau. Les deux premiers acides aminés cités peuvent être utilisés tels quels ou sous la forme de leurs polymères correspondants (peptides). Selon un mode préféré de réalisation de la présente invention, le céramide inhibiteur de la captation du glucose est la N-oléyldihydrosphingosine.

Selon un mode particulièrement préféré de mise en oeuvre du procédé de traitement selon l'invention, on administre en outre à l'organisme, à coté desdits inhibiteurs de la captation du glucose selon l'invention, au moins un composé capable de stimuler la lipolyse. L'apport dudit composé peut alors se faire par voie topique et/ou systémique, et ceci de manière simultanée, séparée, ou encore étalée dans le temps, par rapport à l'étape d'administration des inhibiteurs de la captation du glucose.

Selon un autre aspect de la présente invention, il est également proposé différentes compositions cosmétiques et/ou pharmaceutiques à action amincissante dans les diverses variantes d'administration (voie topique, voie systémique). La présente demande se rapporte donc également aux compositions cosmétiques et/ou dermatologiques à action amincissante comprennant, dans un support physiologiquement acceptable et compatible avec le mode d'administration retenu, au moins une substance non soufrée capable de limiter ou d'inhiber la captation du glucose par les adipocytes, choisie, seule ou en mélanges, parmi la sérine ou ses polymères correspondants, la rutine ou ses polymères correspondants, et les céramides, en association avec au moins un composé capable de stimuler la lipolyse.

Selon un autre aspect de la présente invention, il est également proposé des dispositifs à plusieurs compartiments ou "kits" destinés à la mise en oeuvre du procédé ci-dessus, et qui sont caractérisés par le fait qu'ils comprennent dans un premier compartiment un ou plusieurs inhibiteurs de la captation du glucose, choisi, seul ou en mélanges, parmi la sérine ou ses polymères correspondants, la rutine ou ses polymères correspondants, et les céramides, et, dans un deuxième compartiment, un ou plusieurs stimulateurs de lipolyse, les compositions contenues dans lesdits premier et second compartiments étant ici considérées comme compositions de combinaison pour une utilisation simultanée, séparée ou étalée dans le temps dans un traitement destiné à lutter contre l'adiposité et/ou obtenir un effet d'amincissement.

Enfin, l'invention a également pour objet l'utilisation d'une ou plusieurs substances non soufrées capables de imiter ou d'inhiber la captation du glucose par les adipocytes comme principes actifs dans, ou pour la fabrication de, compositions cosmétiques ou thérapeutiques destinées à lutter contre l'adiposité et/ou à obtenir un effet d'amincissement local ou global de tout ou partie du corps, tant humain qu'animal, la dite substance étant choisie, seule ou en mélanges, parmi la sérine ou ses polymères correspondants, la rutine ou ses polymères correspondants, et les céramides.

Mais d'autres caractéristiques, aspects, objets et avantages de l'invention apparaitront encore plus clairement à la lecture de la description qui va suivre, ainsi que des divers exemple concrets, mais nullement limitatifs, destinés à l'illustrer.

Dans cet exposé, on entend par voie topique, toute technique d'administration d'un produit par application directe de ce dernier sur une partie superficielle (ou externe) du corps, telle que la peau, et par voie systémique, toute technique d'administration d'un produit par une voie autre que topique, par exemple orale et/ou parentérale.

De même, on entend plus simplement désigner, dans ce qui suit, par "inhibiteur de la captation du glucose", toute substance non soufrée permettant, *in vivo*, de limiter ou d'inhiber totalement les mécanismes de captation et de transport du glucose dans les cellules adipeuses (adipocytes), en particulier les mécanismes liés à l'activité de l'un au moins des deux transporteurs du glucose connus sous le nom de GLUT-1 (erythrocyte/brain-type) et GLUT-4 (muscle/adipose-type) présents dans les adipocytes. Le caractère inhibiteur, ou l'absence de caractère inhibiteur, d'une substance donnée vis à vis de la captation du glucose par les adipocytes peut être déterminé par l'homme de l'art au moyen notamment du test biochimique exposé ci-après dans les exemples. Selon la présente invention, on met de préférence en oeuvre des inhibiteurs qui agissent plus spécifiquement ou sélectivement sur GLUT-4, car ce transporteur du glucose est non dépendant de l'insuline, ce qui élimine tout risque d'interférence avec la régulation physiologique de la glycémie.

Enfin, on entend par "stimulateur" de lipolyse, toute substance qui, *in vivo,* permet, directement ou indirectement, de stimuler l'activité de lipolyse dans les adipocytes. De tels agents à activité lipolytique, ainsi que les différents mécanismes d'action qui leur sont associés, sont déja bien connus en soi, et parmi ceux-ci, on peut plus particulièrement citer les bases xanthiques (i.e. des dérivés de la xanthine), telles que la théophylline, la caféine, la théobromine et les 1-hydroxyalkylxanthines et leurs sels compatibles (voir notamment à ce sujet le document FR-A- 2 617 401), les dérivés de l'acide nicotinique tels que plus particulièrement le nicotinate d'alpha-tocophérol et le nicotinate d'hexyle (voir notamment à ce sujet le document EP-A- 371 844), les substances dites alpha-2 bloqueurs capables de bloquer les récepteurs alpha-2 à la surface des adipocytes comme par exemple le ginkgo biloba (voir notamment à ce sujet le document FR-A- 2 669 537), et enfin les facteurs de croissance (voir notamment à ce sujet le document FR-A- 2 671 487), tous ces composés pouvant, dans le cadre de la présente invention, être bien entendu utilisés seuls ou en mélanges.

On notera que, dans le cadre de la présente invention, il est bien évidemment tout à fait possible d'utiliser des mélanges d'inhibiteurs. Par ailleurs, ces inhibiteurs peuvent être apportés sous la forme de produits naturels les contenant, ou bien encore sous la forme de produits synthétiques, lorsque cela est possible.

De manière préférentielle, les inhibiteurs de la captation du glucose sont apportés à l'organisme par voie topique. Cette manière d'opérer permet en effet d'obtenir l'effet d'amincissement recherché de façon beaucoup mieux localisée et controlée (sélectivité) au niveau des différentes parties de la peau que l'on souhaite plus spécifiquement traiter, et ceci par opposition à un mode d'administration par voie systémique, dans lequel l'effet d'amincissement obtenu se trouve généralement globalisé à l'ensemble du corps. Dans le cas où un apport par voie systémique est néanmoins désiré, on préférera alors retenir l'apport par voie orale.

Les mêmes considérations s'appliquent aux stimulateurs de lipolyse, lorsque ces composés sont mis en oeuvre en combinaison (simultanée, séparée ou étalée dans le temps) avec les inhibiteurs de la captation du glucose, c'est à dire qu'ils sont également de préférence administrés par voie topique.

Ainsi, et d'une manière générale, les inhibiteurs de la captation du glucose, ainsi que les éventuels stimulateurs de lipolyse, mis en oeuvre dans le cadre de la présente invention peuvent être classiquement conditionnés sous une forme convenant au mode d'administration ou d'application retenus finalement pour ces derniers (lotions, émulsions, gels, crêmes, comprimés, gélules, dragées, capsules, sirops et autres). Les compositions plus particulièrement visées par la présente invention sont donc des compositions de type cosmétique et/ou pharmaceutique contenant dans un support physiologiquement acceptable au moins un inhibiteur de la captation du glucose à titre de principe actif, en association éventuellement avec au moins un stimulateur de lipolyse, lesdites compositions étant formulées et conditionnées de préférence sous une forme adaptée à une application par voie topique, ou bien encore sous une forme adaptée à une administration par voie systémique, avantageusement orale. Les mêmes considérations s'appliquent au cas des "kits" conformes à l'invention; en particulier, les compositions rentrant dans chacun des compartiments du kit sont de préférence formulées sous une forme convenant à une application topique. A cet égard, on notera que selon la présente invention, il est en fait possible de concevoir des kits de présentation contenant autant de compartiments séparés que de substances actives (inhibiteurs et stimulateurs) que l'on désire ou qu'il est souhaitable de mettre en oeuvre.

Les compositions selon l'invention, ou les kits selon l'invention, ou la mise en oeuvre du procédé selon l'invention, peuvent également faire appel aux divers additifs classiques qui sont utilisés dans les domaines ci-dessus, en particulier aux additifs cosmétiques dans le cas d'applications topiques (produits de soin pour la peau notamment), et choisis par exemple parmi des filtres UV, des agents épaississants, des agents de pénétration tels que l'urée et les alpha-hydroxyacides, des solvants organiques tels que l'éthanol, l'isopropanol, les alkylèneglycols, des agents tensio-actifs choisis parmi les tensio-actifs nonioniques tels que les alkylpolyglycosides, les tensio-actifs cationiques, les tensioactifs anioniques et les tensio-actifs amphotères, des agents solubilisants, des émollients, des colorants, des parfums, des conservateurs et, d'une manière générale, tous les excipients usuels rencontrés dans le domaine de la pharmacopée.

Il est bien entendu également possible d'intégrer dans les compositions selon l'invention des produits (autres que des stimulateurs de lipolyse) déja connus en soi comme présentant une activité plus ou moins marquée dans le domaine de la lutte contre l'adiposité et/ou l'amincissement, comme par exemple certains extraits végétaux de type huileux, hydrosolubles ou hydro-alcooliques. Parmi ceux-ci, on peut plus particulièrement citer l'extrait de lierre grimpant (Hedera Helix), d'arnica (Arnica Montana L), de romarin (Rosmarinus officinalis N), de souci (Calendula officinalis), de sauge (Salvia officinalis L), de ginseng (Panax ginseng), de millepertuis (Byperycum Perforatum), de fragon (Ruscus aculeatus L), d'ulmaire (Filipendula ulmaria L), d'orthosiphon (Orthosiphon Stamincus Benth), d'algues (Fucus Vesiculosus), de bouleau (Betula alba), de noix de cola (Cola Nipida), tous ces extraits pouvant bien entendu être pris en mélanges.

Les quantités d'inhibiteurs présentes dans les compositions selon l'invention ne sont pas critiques et peuvent ainsi varier dans de très larges limites, lesquelles sont en particulier fonctions du mode de présentation et/ou d'administration retenu pour ces dernières. En particulier, on peut, si on le désire, utiliser les produits purs. Dans le cas par exemple de préparations à usage topique, cette quantité peut ainsi aller sans problèmes de 0,0001% en poids à 100% en poids par rapport à l'ensemble de la composition, de préférence entre 0,5% et 10% en poids. Dans le cas de préparations destinées à une administration par voie systémique, les doses doivent rester compatibles avec les impératifs classiques liés à la toxicologie et la galénique des produits pharmaceutiques; à cet égard, des doses d'administration comprises entre 1,µg/10Kg (du poids du sujet traité)/jour et 15g/10Kg/jour conviennent généralement.

Pour obtenir des effets notables, les fréquences d'administration ou d'application des compositions selon l'invention, qui peuvent être bien entendu variables selon les quantités d'agents inhibiteurs mises en oeuvre à chaque opération, sont de l'ordre de une à deux fois par jour. Le traitement est ensuite poursuivi régulièrement, pendant plusieurs jours, de préférence pendant plusieurs semaines, voire plusieurs mois. Il n'y a en fait aucun inconvénient ou contre-indication à pratiquer continuellement et quotidiénement sur le corps le traitement selon l'invention. Dans le cas d'une administration par voie topique, l'application des compositions s'accompagne avantageusement d'un massage simultané de la partie du corps traitée.

On a noté que les quantités suffisamment efficaces en agents inhibiteurs à mettre en oeuvre dans le cadre de la présente invention peuvent, généralement, rester très faibles.

La présente invention trouve ainsi des applications particulièrement utiles et intéressantes dans le domaine des traitements cosmétiques visant à obtenir des effets locaux ou généralisés d'amincissement et/ou affinement de la peau ou de la silhouette (hanches, fesses, cuisses, ventre et autres) ou dans le domaine des traitements des diverses pathologies qui sont liées à l'adiposité, en particulier l'obésité.

L'un des grands avantages de la présente invention réside dans la possibilité de pouvoir procéder, chaque fois que nécessaire ou souhaitable, à des traitements "doux" très localisés et sélectifs grâce au mode d'application par voie topique.

La présente invention est bien entendu applicable au corps tant humain qu'animal.

Des exemples concrets illustrant l'invention vont maintenant être donnés.

### Exemple 1

Cet exemple a pour but de mettre en évidence l'effet *in vitro* de certaines substances sur le transport du glucose dans les adipocytes.

Les substances testées ont été les suivantes :
- la sérine (G1)
- la thréonine (G2)
- la poudre de bifidus (G3)
- la rutine (G4)
- la N-oléyldihydrosphingosine (G5)
- la D-sphingosine (G6)

### 1- Protocole expérimental

1.1- Fixation de 2-déoxyglucose : l'ensemble des opérations se déroule à 37°C. Les cellules préadipocytaires (lignée Ob17) sont différenciées en culture dans un milieu contenant 8% de sérum de boeuf, 17 nM d'insuline, 2 nM de triidothyronine et 2 nM de somatotropine (milieu de différenciation). Une fois différenciées, les cellules, qui sont et restent attachées au fond de la boite en culture (puits de 16 mm), sont maintenues en présence de 1 ml de milieu DME supplémenté avec 8% de sérum de veau fétal, en l'absence ou en présence de 100 nM insuline, et en l'absence ou en présence des substances G1 à G6 ci-dessus à diverses concentrations. Après 48 heures, le milieu est éliminé et la même opération renouvelée pendant 48h. L'exposition chronique aux substances G1-G6 dure donc au total 4 jours.
   Ensuite, les cellules sont lavées pendant 1h30 avec du milieu DME et préincubées pendant 30 mn dans 1 ml de tampon à pH 7,4 contenant 20 mM Hepes, 140 mM NaCI, 1 mM CaCl₂, 5 mM KCl, 2,5 mM MgSO₄ et 2% BSA-fatty acid poor (tampon KRP/BSA).
   Au temps zéro, 10 µl de [³H]2-déoxyglucose (2-DOG) (0,1 mM final ; 1µCi/boîte) sont ajoutés. Chaque point de la cinétique (0 - 5 - 15 mn) est tripliqué (3 puits séparés par point). La réaction est arrétée par trois lavages rapides avec du "phosphate-buffered saline" (PBS) pH 7,4 à 4°C contenant 0,1 mM DOG non radioactif. Les cellules sont ensuite lysées dans 1 ml de soude 0,1 N. Un aliquot de 0,9 ml est prélevé pour la mesure de la radioactivité incorporée et 0,1 ml est utilisé pour le dosage de protéines. Les résultats sont exprimés en moyenne +/- SEM (pmoles de 2-DOG incorporés/min/mg de protéine).
1.2- Viabilité : technique d'exclusion du Bleu Trypan et relarguage de lactate déshydrogénase.
1.3- Synthèse protéique : les cellules sont traitées comme décrit au point 1.1 ci-dessus et incubées à 37°C en présence de 1 ml de tampon KRP contenant [³H]leucine (10 µM final ; 0,5 µCi par puits). Après 2 mn, la réaction est arrêtée par lavage avec du tampon KRP contenant 10 µM leucine non radioactive. La radioactivité des cellules est mesurée, et les résultats exprimés en pmoles/mn/mg protéine (moyenne +/- SEM). Les valeurs des cellules contrôles comme des cellules traitées chroniquement par les molécules G1 à G6 sont restées pratiquement identiques (40 +/-4 pmoles/mn/mg protéine).

### 2- Résultats

Le transport "basal" est celui observé dans des cellules différenciées maintenues pendant 4 jours en l'absence d'insuline et avec des concentrations croissantes des diverses substances G1 à G6.

Le transport "insulino-stimulé" est celui observé dans des cellules différenciées maintenues pendant 4 jours en la présence de 100 nM insuline et avec des concentrations croissantes des diverses substances G1 à G6.

Les résultats montrent que l'uptake de 2-DOG est très significativement augmenté dans les cellules exposées chroniquement (4 jours) à l'insuline. Comme attendu, le transport est totalement inhibé par 10 µM cytochalasine B, que les cellules aient été ou non exposées à l'insuline.

Les résultats montrent par ailleurs que le transport "insulino-stimulé" n'est diminué que faiblement par exposition chronique aux diverses substances G1 à G6, alors que le transport "basal" diminue significativement par exposition chronique à 10 µg/ml des substances G1 (- 30%),G4 (- 30%), et G5 (- 35%).

### 3- Conclusions

Les diverses substances G1 à G6 ne présentent pas de caractère de cytotoxicité aux concentrations utilisées. L'effet inhibiteur est observé pour certaines substances données (G1, G4 et G5) en chronique sur le transport de 2-DOG dans des cellules qui n'ont subi aucun traitement à l'insuline ; par contre, les substances G2 (proche de G1), G3, et G6 (proche de G5) ne donnent aucun effet notable. Les effets chroniques des substances spécifiques G1, G4 et G5 sur la diminution du transport du 2-DOG dans les cellules non exposées à l'insuline passent vraissemblablement en priorité par un effet sur GLUT-4 et par une absence d'effet sur GLUT-1; sans vouloir limiter la présente invention à une quelconque théorie, cet effet sur GLUT-4 pourrait alors se produire soit par une diminution de la synthèse de ce transporteur, soit par une diminution de la proportion de GLUT-4 présent à la surface cellulaire sans modification de leur synthèse.

### Exemple 2

Cet exemple a pour but d'illustrer diverses formulations concrètes, de type cosmétique, rentrant dans le cadre de la présente invention.

### A) Baume (voie topique) :

| | |
|---|---|
| - Ozokérite | 10 g |
| - Palmitate d'isopropyle | 10 g |
| - Vaseline blanche | 15 g |
| - Conservateur | 0,2 g |
| - Antioxydants | 0,3 g |
| - Parfum | 1 g |
| - N-oléyldihydrosphingosine | 1 g |
| - Huile de vaseline qsp | 100 g |

### B) Baume (voie topique) :

| | |
|---|---|
| - Ozokérite | 20 g |
| - Huile de purcellin liquide | 10 g |
| - Vaseline Blanche | 15 g |
| - Conservateur | 0,2 g |
| - Antioxydant | 0,3 g |
| - N-oléyldihydrosphingosine | 1 g |
| - nicotinate d'α-tocophérol | 0,1 g |
| - Huile de vaseline qsp | 100 g |

### C) Gel émulsionné de type H/E (voie topique) :

| | |
|---|---|
| - Carbopol® 940 (commercialisé par Goodrich) | 0,6 g |
| - Huile de silicone volatile | 3 g |
| - Huile de purcellin | 7 g |
| - Tefose® 63 | 3 g |
| - Conservateur | 0,3 g |
| - Alcool éthylique | 15 g |
| - Parfum | 0,4 g |
| - Triéthanolamine | 0,2 g |
| - Rutine | 0,2 g |
| - Caféine | 3 g |
| - Eau déminéralisée qsp | 100 g |

### D) Gel hydroalcoolique (voie topique) :

| | |
|---|---|
| - Carbopol® 941 (commercialisé par Goodrich) | 1 g |
| - Triéthanolamine | 1 g |
| - Ethanol 95% | 60 g |
| - Glycérol | 3 g |
| - Propylèneglycol | 2 g |
| - Sérine | 0,3 g |
| - Aescine | 0,5 g |
| - Eau déminéralisée qsp | 100 g |

### E) Gel anhydre (voie topique) :

| | |
|---|---|
| - Ethanol absolu | 61 g |
| - Hydroxyéthylcellulose | 0,8 g |
| - Propylèneglycol | 25 g |
| - Polyéthylèneglycol | 12 g |
| - N-oléyldihydrosphingosine | 0,2 g |

### F) Emulsion de type H/E (voie topique) :

| | |
|---|---|
| - Huile de silicone volatile | 10 g |
| - Perhydrosqualène | 18 g |
| - Huile de vaseline | 5 g |
| - Lanoline liquide | 4 g |
| - Arlacel® 165 (commercialisé par Atlas) | 6 g |
| - Tween® 60 (commercialisé par Atlas) | 2 g |
| - Alcool cétylique | 1,2 g |
| - Acide stéarique | 2,5 g |
| - Triéthanolamine | 0,1 g |
| - Conservateur | 0,3 g |
| - Antioxydants | 0,3 g |
| - Rutine | 1 g |
| - Acide lactique | 0,5 g |
| - Eau déminéralisée qsp | 100 g |

### G) Emulsion de type H/E (voie topique) :

| | |
|---|---|
| - Propylèneglycol | 2 g |
| - PEG 400 | 3 g |
| - Conservateur | 0,3 g |
| - Carbopol® 941 | 0,2 g |
| - Myristate d'isopropyle | 1 g |
| - Alcool cétylique | 3g |
| - Acide stéarique | 3 g |
| - Monostéarate de glycérol | 3 g |
| - Huile de germe de maïs | 2 g |
| - Parfum | 0,5 g |
| - Sérine | 0,2 g |
| - Eau déminéralisée qsp | 100 g |

### H) Gel limpide (voie topique) :

| | |
|---|---|
| - Nonylphénol oxyéthyléné (à 12 moles d'oxyde d'éthylène) | 5 g |
| - Carbopol® 940 | 1 g |
| - Alcool éthylique | 30 g |
| - Triéthanolamine | 0,3 g |
| - Glycérine | 3 g |
| - Parfum | 0,3 g |
| - Conservateur | 0,3 g |
| - Sérine | 0,5 g |
| - Eau déminéralisée qsp | 100 g |

### I) Crème aux Liposomes (voie topique) :

| | |
|---|---|
| - Alcool cétylique polyglycérolé | 3,8 g |
| - B- sitostérol | 3,8 g |
| - Dicétyl phosphate | 0,4 g |
| - Conservateur | 0,3 g |
| - Huile de tournesol | 35 g |
| - Parfum | 0,6 g |
| - Carbopol® 940 | 0,2 g |
| - Triéthanolamine | 0,2 g |
| - N-oléyldihydrosphingosine | 0,05 g |
| - Sérine | 0,5 g |
| - Caféine | 1 g |
| - Eau déminéralisée qsp | 100 g |

### J) Gélules (voie orale) :

| | |
|---|---|
| - Aérosil® 200 (silice) | 5 mg |
| - Stéarate de zinc | 5 mg |
| - Talc | 5 mg |
| - Sérine | 200 mg |
| - Lactose qsp | 400 mg |

Toutes les formulations A)-I) ci-dessus, après application répétée sur la peau, ont permis d'obtenir un effet d'amincissement notable chez des individus atteints d'adiposité locale. Le même effet a été observé après administration répétée par voie orale de la composition J).

## Revendications

1. Procédé de traitement non thérapeutique pour lutter contre l'adiposité, caractérisé en ce qu'il consiste à administrer à l'organisme, par voie topique et/ou systémique, au moins une substance non soufrée capable de limiter ou d'inhiber la captation du glucose par les adipocytes, choisie, seule ou en mélanges, parmi la sérine ou ses polymères correspondants, la rutine ou ses polymères correspondants, et les céramides.

2. Procédé selon la revendication 1, caractérisé en ce que ladite substance est administrée par voie topique.

3. Procédé selon la revendication 1, caractérisé en ce que ladite substance est administrée par voie orale.

4. Procédé selon l'une quelconque des revendications 1 à 3, caractérisé en ce que ladite substance est la N-oléyldihydrosphingosine.

5. Procédé selon l'une quelconque des revendications précédentes, caractérisé en ce que l'on apporte en outre à l'organisme, par voie topique et/ou systémique, au moins un composé capable de stimuler la lipolyse.

6. Procédé selon la revendication 5, caractérisé en ce que ledit apport dudit stimulateur se fait de manière simultanée, séparée ou étalée dans le temps, par rapport à l'étape d'administration des inhibiteurs de la captation du glucose.

7. Procédé selon l'une des revendications 5 ou 6, caractérisé en ce que ledit apport se fait par voie topique.

8. Procédé selon la revendications 7, caractérisé en ce qu'il consiste à utiliser une composition à usage topique contenant à la fois les inhibiteurs de la captation du glucose d'une part et les stimulateurs de lipolyse d'autre part.

9. Procédé selon l'une quelconque des revendications 5 à 8, caractérisé en ce que l'on utilise des stimulateurs de lipolyse qui sont choisis, seuls ou en mélanges, parmi les bases xanthiques, en particulier la théophylline, la caféine, la théobromine et les 1-hydroxyalkylxanthines et leurs sels compatibles, les dérivés de l'acide nicotinique tels que plus particulièrement le nicotinate d'alphatocophérol et le nicotinate d'hexyle, les substances dites alpha-2 bloqueurs, en particulier le ginkgo biloba, et les facteurs de croissance.

10. Procédé selon l'une quelconque des revendications précédentes, pour l'obtention d'un effet d'amincissement et/ou d'affinement de la peau et/ou de la silhouette.

11. Composition cosmétique et/ou pharmaceutique à action amincissante, caractérisée en ce qu'elle comprend, dans un support physiologiquement acceptable, au moins une substance non soufrée capable de limiter ou d'inhiber la captation du glucose par les adipocytes, choisie, seule ou en mélanges, parmi la sérine ou ses polymères correspondants, la rutine ou ses polymères correspondants, et les céramides, et au moins un stimulateur de lipolyse.

12. Composition selon la revendication 11, caractérisée en ce que ladite substance est telle définie à la revendication 4.

13. Composition selon la revendication 11 ou 12, caractérisée en ce que ledit stimulateur de lipolyse est tel que défini à la revendication 9.

14. Composition selon l'une quelconque des revendications 11 à 13, caractérisée en ce qu'elle est conditionnée sous une forme convenant à une administration par voie systémique, de préférence orale.

15. Composition selon l'une quelconque des revendications 11 à 13, caractérisée en ce qu'elle est conditionnée sous une forme convenant à une application par voie topique.

16. Dispositif à plusieurs compartiments ou "kit", caractérisé par le fait qu'il comprend dans un premier compartiment au moins un inhibiteur de la captation du glucose, choisie, seule ou en mélanges, parmi la sérine ou ses polymères correspondants, la rutine ou ses polymères correspondants, et les céramides, et, dans un deuxième compartiment, au moins un stimulateur de lipolyse.

17. "Kit" selon la revendication 16, caractérisé en ce que les composés rentrant dans lesdits premier et deuxième compartiments sont conditionnés sous une forme convenant à une application topique.

18. "Kit" selon l'une des revendications 16 ou 17, caractérisé en ce que l'inhibiteur est la N-oléyldihydrosphingosine.

19. "Kit" selon l'une des revendications 16 à 18, caractérisé en ce que le stimulateur est choisi parmi les bases xanthiques, en particulier la théophylline, la caféine, la théobromine et les 1-hydroxyalkylxanthines et leurs sels compatibles, les dérivés de l'acide nicotinique tels que plus particulièrement le nicotinate d'alpha-tocophérol et le nicotinate d'hexyle, les substances dites alpha-2 bloqueurs, en particulier le ginkgo biloba, les facteurs de croissance, et leurs mélanges.

20. Utilisation d'une substance non soufrée capable de limiter ou d'inhiber la captation du glucose par les adipocytes comme principe actif dans, ou pour la fabrication de, une composition cosmétique ou thérapeutique destinée à lutter contre l'adiposité et/ou à obtenir un effet d'amincissement local ou général du corps, ladite substance étant choisie, seule ou en mélanges, parmi la sérine ou ses polymères correspondants, la rutine ou ses polymères correspondants et les céramides.

## Claims

1. Non-therapeutic treatment process for combating adiposity, characterized in that it consists in administering to the body, via the topical and/or systemic route, at least one sulphur-free substance capable of limiting or of inhibiting glucose uptake by adipocytes, the substance being chosen, alone or as mixtures, from serine or its corresponding polymers, rutine or its corresponding polymers, and ceramides.

2. Process according to Claim 1, characterized in that the said substance is administered via the topical route.

3. Process according to Claim 1, characterized in that the said substance is administered via the oral route.

4. Process according to any one of Claims 1 to 3, characterized in that the said substance is N-oleyldihydrosphingosine.

5. Process according to any one of the preceding claims, characterized in that the body is additionally supplied, via the topical and/or systemic route, with at least one compound capable of stimulating lipolysis.

6. Process according to Claim 5, characterized in that the said supply of the said stimulator is made in a simultaneous or separate manner or spread out over time relative to the step for administration of the glucose-uptake inhibitors.

7. Process according to either of Claims 5 and 6, characterized in that the said supply is made via the topical route.

8. Process according to Claim 7, characterized in that it consists in using a composition for topical use containing both the glucose-uptake inhibitors, on the one hand, and the lipolysis stimulators, on the other hand.

9. Process according to any one of Claims 5 to 8, characterized in that lipolysis stimulators are used which are chosen, alone or as mixtures, from xanthic bases, in particular theophylline, caffeine, theobromine and 1-hydroxyalkylxanthines and their compatible salts, nicotinic acid derivatives, more particularly such as alpha-tocopherol nicotinate and hexyl nicotinate, substances known as alpha-2 blockers, in particular ginkgo biloba, and growth factors.

10. Process according to any one of the preceding claims, in order to obtain a slimming and/or thinning effect of the skin and/or of the figure.

11. Cosmetic and/or pharmaceutical composition with slimming action, characterized in that it comprises, in a physiologically acceptable support, at least one sulphur-free substance capable of limiting or of inhibiting glucose uptake by adipocytes, the substance being chosen, alone or as mixtures, from serine or its corresponding polymers, rutine or its corresponding polymers, and ceramides, and at least one lipolysis stimulator.

12. Composition according to Claim 11, characterized in that the said substance is as defined in Claim 4.

13. Composition according to Claim 11 or 12, characterized in that the said lipolysis stimulator is as defined in Claim 9.

14. Composition according to any one of Claims 11 to 13, characterized in that it is packaged in a suitable form for administration via a systemic route, preferably via the oral route.

15. Composition according to any one of Claims 11 to 13, characterized in that it is packaged in a suitable form for application via the topical route.

16. Device containing several compartments or "kit", characterized in that it comprises, in a first compartment, at least one glucose-uptake inhibitor chosen, alone or as mixtures, from serine or its corresponding polymers, rutine or its corresponding polymers, and ceramides, and, in a second compartment, at least one lipolysis stimulator.

17. "Kit" according to Claim 16, characterized in that the compounds included in the said first and second compartments are packaged in a suitable form for topical application.

18. "Kit" according to either of Claims 16 and 17, characterized in that the inhibitor is N-oleyldihydrosphingosine.

19. "Kit" according to one of Claims 16 to 18, characterized in that the stimulator is chosen from xanthic bases, in particular theophylline, caffeine, theobromine and 1-hydroxyalkylxanthines and their compatible salts, nicotinic acid derivatives, more particularly such as alpha-tocopherol nicotinate and hexyl nicotinate, substances known as alpha-2 blockers, in particular ginkgo biloba, growth factors, and mixtures thereof.

20. Use of a sulphur-free substance capable of limiting or of inhibiting glucose uptake by adipocytes as active principle in, or for the production of, a cosmetic or therapeutic composition intended to combat adiposity and/or to obtain a local or general slimming effect on the body, the said substance being chosen, alone or as mixtures, from serine or its corresponding polymers, rutine or its corresponding polymers, and ceramides.

## Patentansprüche

1. Verfahren zur nicht-therapeutischen Behandlung zur Bekämpfung der Adipositas,
**dadurch gekennzeichnet, daß**
es darin besteht, dem Organismus auf topischem und/oder systemischem Wege mindestens eine nicht-schwefelhaltige Substanz zu verabreichen, die befähigt ist, die Glucoseaufnahme durch die Adipocyten zu begrenzen oder zu hemmen, und die, allein oder im Gemisch, unter Serin und seinen entsprechenden Polymeren, Rutin und seinen entsprechenden Polymeren und Ceramiden ausgewählt wird.

2. Verfahren nach Anspruch 1, dadurch gekennzeichnet, daß die Substanz topisch verabreicht wird.

3. Verfahren nach Anspruch 1, dadurch gekennzeichnet, daß die Substanz oral verabreicht wird.

4. Verfahren nach einem der Ansprüche 1 bis 3, dadurch gekennzeichnet, daß es sich bei der Substanz um N-Oleyldihydrosphingosin handelt.

5. Verfahren nach einem der vorhergehenden Ansprüche, dadurch gekennzeichnet, daß dem Organismus auf topischem und/oder systemischem Wege mindestens eine Verbindung zur Stimulierung der Lipolyse zugeführt wird.

6. Verfahren nach Anspruch 5, dadurch gekennzeichnet, daß die Zugabe der stimulierenden Verbindung gleichzeitig, getrennt oder über einen längeren Zeitraum, bezogen auf die Verabreichung der die Glucoseaufnahme hemmenden Substanz, erfolgt.

7. Verfahren nach einem der Ansprüche 5 und 6, dadurch gekennzeichnet, daß die Zugabe topisch erfolgt.

8. Verfahren nach Anspruch 7, dadurch gekennzeichnet, daß es in der Verwendung einer topisch zu verabreichenden Zusammensetzung besteht, die sowohl die die Glucoseaufnahme hemmenden Substanzen als auch die die Lipolyse stimulierenden Verbindungen enthält.

9. Verfahren nach einem der Ansprüche 5 bis 8, dadurch gekennzeichnet, daß die die Lipolyse stimulierenden Verbindungen, allein oder im Gemisch, ausgewählt werden unter Xanthinbasen, insbesondere Theophyllin, Coffein und Theobromin, und 1-Hydroxyalkylxanthinen und ihren kompatiblen Salzen, Nicotinsäurederivaten, wie z.B. insbesondere α-Tocopherylnicotinat, Hexylnicotinat, den als α-2-Blocker bezeichneten Substanzen insbesondere Ginkgo biloba, und den Wachstumsfaktoren.

10. Verfahren nach einem der vorhergehenden Ansprüche zum Erhalt einer schlankmachenden Wirkung und/oder Verbesserung der Haut und/oder der Figur.

11. Kosmetische und/oder pharmazeutische Zusammensetzung mit schlankmachender Wirkung, dadurch gekennzeichnet, daß sie in einem physiologisch akzeptablen Träger mindestens eine nicht-schwefelhaltige Substanz enthält, die befähigt ist, die Glucoseaufnahme durch die Adipocyten zu begrenzen oder zu hemmen, die, allein oder im Gemisch, ausgewählt wird unter Serin und seinen entsprechenden Polymeren, Rutin und seinen entsprechenden Polymeren und Ceramiden, und mindestens eine die Lipolyse stimulierende Verbindung enthält.

12. Zusammensetzung nach Anspruch 11, dadurch gekennzeichnet, daß die Substanz wie in Anspruch 4 definiert ist.

13. Zusammensetzung nach Anspruch 11 oder 12, dadurch gegekennzeichnet, daß es sich bei der die Lipolyse stimulierenden Verbindung um eine der in Anspruch 9 beschriebenen Verbindungen handelt.

14. Zusammensetzung nach einem der Ansprüche 11 bis 13, dadurch gekennzeichnet, daß sie so formuliert ist, daß sie systemisch und vorzugsweise oral verabreicht werden kann.

15. Zusammensetzung nach einem der Ansprüche 11 bis 13, dadurch gekennzeichnet, daß sie so formuliert ist, daß sie topisch verabreicht werden kann.

16. Vorrichtung mit mehreren Abteilungen oder Kit, dadurch gekennzeichnet, daß sie in einer ersten Abteilung mindestens eine die Glucoseaufnahme hemmende Substanz enthält, die, allein oder im Gemisch, unter Serin und seinen entsprechenden Polymeren, Rutin und seinen entsprechenden Polymeren und Ceramiden ausgewählt ist, und in einer zweiten Abteilung mindestens eine die Lipolyse stimulierende Verbindung enthält.

17. Kit nach Anspruch 16, dadurch gekennzeichnet, daß die in der ersten und zweiten Abteilung enthaltenen Zusammensetzungen so formuliert sind, daß sie topisch verabreicht werden können.

18. Kit nach einem der Ansprüche 16 oder 17, dadurch gekennzeichnet, daß es sich bei der hemmenden Substanz um N-Oleyldihydrosphingosin handelt.

19. Kit nach einem der Ansprüche 16 bis 18, dadurch gekennzeichnet, daß die stimulierende Verbindung ausgewählt ist unter Xanthinbasen, insbesondere Theophillin, Coffein und Theobromin, und 1-Hydroxyalkylxanthinen und ihren kompatiblen Salzen, den Nicotinsäurederivaten, wie z.B. insbesondere α-Tocopherylnicotinat und Hexylnicotinat, den als α-2-Blockern bezeichneten Substanzen, insbesondere Ginkgo biloba, den Wachstumsfaktoren und deren Gemischen.

20. Verwendung einer nicht-schwefelhaltigen Substanz, die befähigt ist, die Glucoseaufnahme durch die Adipocyten zu begrenzen oder zu hemmen, als ersten Wirkstoff in einer kosmetischen oder therapeutischen Zusammensetzung, die zur Bekämpfung der Adipositas und/oder der Erzielung einer lokalen oder allgemeinen schlankmachenden Wirkung auf den Körper bestimmt ist, oder für deren Herstellung, wobei die Substanz, allein oder im Gemisch, ausgewählt ist unter Serin und seinen entsprechenden Polymeren, Rutin und seinen entsprechenden Polymeren und Ceramiden.
